# EUROPÄISCHE PATENTSCHRIFT

(11) **EP 0 712 943 B1**
(45) Veröffentlichungstag und Bekanntmachung des Hinweises auf die Patenterteilung: **18.06.2003**
(21) Anmeldenummer: 95250199.7
(22) Anmeldetag: 15.08.1995
(51) Int. Cl.: C23C 16/32, C23C 16/02, A61L 31/00, A61L 27/00, A61L 33/00

(54) **Verfahren zur Herstellung einer nichtkollabierenden intravasalen Gefässprothese (Stent)**
Method for making non-collapsible intravascular prosthesis (stent)
Procédé pour fabriquer une prothèse intravasculaire qui ne s'affaisse pas (stent)

(30) Priorität: 15.08.1994 DE 4429380
(43) Veröffentlichungstag der Anmeldung: 22.05.1996
(73) Patentinhaber: BIOTRONIK Mess- und Therapiegeräte GmbH & Co Ingenieurbüro Berlin, 12359 Berlin (DE)
(72) Erfinder: Amon, Michael, D-91077 Dormitz (DE); Bolz, Armin, D-91058 Erlangen (DE)
(74) Vertreter: Eisenführ, Speiser & Partner

(56) Entgegenhaltungen:
- EP-A- 0 601 804
- GB-A- 2 165 266
- IMAGES OF THE TWENTY-FIRST CENTURY. PROCEEDINGS OF THE ANNUAL INTERNATIONAL CONFERENCE OF THE IEEE ENGINEERING IN MEDICINE AND BIOLOGY SOCIETY (CAT. NO.89CH2770-6), SEATTLE, WA, USA, 9-12 NOV. 1989, Bd. 11, 1989, NEW YORK, NY, USA, IEEE, USA, Seiten 164-166 , BOLZ A ET AL 'New coating materials for artificial heart valves'
- PATENT ABSTRACTS OF JAPAN vol. 006 no. 261 (C-141) ,21.Dezember 1982 & JP-A-57 155365 (MITSUBISHI KINZOKU KK) 25.September 1982,

## Beschreibung

Die Erfindung betrifft ein Verfahren zur Herstellung einer Gefäßprothese der im Oberbegriff des Anspruchs 1 angegebenen Art.

Kardiovaskuläre Implantate sind insbesondere als Herzklappen- oder Gefäßprothesen oder Herzschrittmacherelektroden im menschlichen Körper einsetzbar. Dabei kommt es auf hohe Blutvertraglichkeit (Antithrombogenität) der Implantatoberfläche an.

Ein Vakuumbeschichtungsverfahren zur Bildung einer ultradünnen Polymerschicht auf einem Kunststoff-Implantat zur Erhöhung der Biokompatibilität beschreibt US 4,656,083.

Die plasmaunterstützte chemische Gasphasenabscheidung einer blutverträglichen SiC-Schicht auf einem kardiovaskulären Implantat, nämlich einer Herzklappe, betrifft der Artikel "New Coating Materials for Artificial Heart Valves" von Bolz et al. (Images of the Twenty-First Century; Proceedings of the Annual International Conference of the IEEE Engineering in Medicine and Biology Society, Seattle, WA, USA, 9-12 Nov. 1989, Bd. 11, 1989, New York, NY, USA, IEEE, USA, Seite 164-166).

Die Beschichtung von metallischem Substraten mit Halbleiterverbindungsschichten wie SiC ist im übrigen hinlänglich bekannt, beispielsweise aus der JP-A-57 155 365, bei der die Beschichtung des Substrats mittels Funkenentladung erfolgt.

Hinsichtlich der Blutverträglichkeit haben gattungsgemäße Implantate, wie sie in EP 0 371 908 B1 beschrieben sind und die eine Beschichtung etwa aus Siliziumcarbid (SiC) - insbesondere mit amorpher Schichtstruktur - aufweisen, weitgehend befriedigende Parameter erreicht. Diese Beschichtungen können in einfacher Weise und hoher Qualität durch ein Verfahren der plasmaunterstützten chemischen Gasphasenabscheidung (PECVD = plasma enhanced chemical vapour deposition) hergestellt werden, wie in US 5,238,866 ausführlich beschrieben.

Die Bildung der Beschichtung auf dem Träger erfolgt jedoch bei relativ hohen Temperaturen um 250°C , u.U. bis zu 350°C, so daß bei der Abkühlung Gefügespannungen entstehen, die das Haftvermögen der Beschichtung begrenzen.

Im Zusammenhang mit der in den letzten Jahren praktizierten und methodisch intensiv vorangetriebenen perkutanen transluminalen Koronarangioplastie (PTCA) hat die Verwendung von intravasal aufweitbaren, nichtkollabierenden Gefäßprothesen (Stents) Bedeutung erlangt.

Bei solchen Implantaten, die einen streckmetallartigen Wandungsaufbau haben, der im Gebrauch einer erheblichen Verformung des Materials unterworfen wird, kommt es in bisher nicht gekanntem Maße auf eine ausreichende Gleichmaßdehnung, d.h. gute plastische Verformbarkeit bei geeigneter Streckgrenze, an. Dies erfordert eine außerordentlich starke Haftung zwischen dem Träger und der biokompatiblen Beschichtung, wobei die Haftung eine Ablösung der Beschichtung auch bei relativ starken Verformungen des Trägers verhindern muß.

Der Erfindung liegt daher die Aufgabe zugrunde, ein Verfahren zur Herstellung einer Gefäßprothese der eingangs genannten Gattung bereitzustellen, mit dem ein Stent mit blutverträglicher Oberflächenschicht hergestellt werden kann, die eine sehr gute Haftung auf dem Trägermaterial, insbesondere Edelstahl, aufweist.

Diese Aufgabe wird durch ein Verfahren nach Anspruch 1 gelöst.

Die Erfindung schließt den Gedanken ein, vor dem Aufbringen der biokompatiblen Beschichtung die Oberfläche des Trägers gründlich von Adsorbaten (insbesondere Kohlenwasserstoffen und Oxiden) zu reinigen. Sie schließt weiterhin den Gedanken ein, eine Anreicherung von Fehlstellen in der Trägeroberfläche herbeizuführen, um günstige Voraussetzungen für eine stabile chemische und grenzflächenphysikalische Anbindung der aufzubringenden Beschichtung zu schaffen. Schließlich schließt sie den Gedanken ein, dazu die zur Schichtbildung ohnehin erforderliche Anlage zu nutzen.

Eine in vorteilhafter Weise weiter erhöhte Haftfestigkeit kann erzielt werden, indem nach der Oberflächenbehandlung des vorgefertigten Stents ein Zwischenschritt ausgeführt wird. Dieser umfaßt das Anlegen einer vorbestimmten zweiten negativen Biasspannung an den oberflächenbehandelten Stent, das Zuführen eines das Halbleitermaterial in gebundener Form enthaltenden Prozeßgases mit einer vorbestimmten Flußrate und das Einkoppeln von HF-Leistung mit einer vorbestimmten Leistungsdichte in den Sputter-Reaktor für eine vorbestimmte Zeitdauer zur Ausführung einer plasmagestützten Abscheidung einer dünnen, das Halbleiterelement aufweisenden Haftvermittlerschicht auf den Stent.

Vorzugsweise ist das Halbleiter-Element Silizium, und die Halbleiterverbindungsschicht weist Siziumkarbid auf bzw. besteht im wesentlichen daraus. Insbesondere wird die Schicht als amorphe Schicht gebildet. Ihre Dicke beträgt vorzugsweise einige hundert, insbesondere etwa 400, nm.

Als Ätzgas im Schritt der Oberflächenbehandlung wird vorzugsweise ein Edelgas, insbesondere Argon, Stickstoff oder Tetrachlorkohlenstoff verwendet, während das Gemisch aus Prozeßgasen im Schritt der Gasphasenabscheidung Monosilan und Methan aufweist.

Die Haftvermittlerschicht besteht bei Verwendung einer Siliziumverbindungs-Deckschicht im wesentlichen aus Silizium, wobei auch das Prozeßgas im Schritt der Bildung der Haftvermittlerschicht zweckmäßigerweise Monosilan sein kann, und wird einige, insbesondere 3 bis 5 nm, dick gebildet.

Als Träger für die Beschichtung wird ein vorgefertigter Stent vorzugsweise aus Edelstahl, alternativ aber auch einer Titan- oder Tantallegierung oder Platin/Iridium, verwendet.

Wichtig für eine gute Haftung der Beschichtung auf dem Träger ist, daß die Temperatur des vorgefertigten Stents während des gesamten Prozeßablaufes konstant, vorzugsweise auf im wesentlichen 250 °C, gehalten wird. Hierdurch wird die Entstehung von Schichtspannungen während des Prozeßablaufes verhindert. Zu diesem Zweck wird beim Übergang zum Schritt der Gassphasenabscheidung und bei Ausführung dieses Schritts der Stent über eine im wesentlichen trägheitslose Substratheizung beheizt, da in dieser Phase im wesentlichen keine Aufheizung mehr durch einen Ionenbeschuß erfolgt.

Um zwischen den Prozeßschritten eine Anlagerung von Restgasen an die Stent-Oberfläche zu verhindern, erfolgt in vorteilhafter Weise die Trennung des Stents von der negativen Biasspannung ohne Unterbrechung der Einkopplung der HF-Leistung und somit unter Aufrechterhaltung des Plasmas im Rezipienten.

Die Haftvermittlerschicht und die Halbleiterverbindungsschicht können durch kontinuierliche Veränderung der Prozeßgaszusammensetzung über einen vorbestimmten Zeitraum ohne ausgeprägte Grenzfläche unter Abnahme des Anteils des Halbleiterelementes - etwa des elementaren Si - mit zunehmendem Abstand von der Oberfläche des vorgefertigten Stents ineinander übergehend gebildet werden.

Bei einem mit dem erfindungsgemäßen Verfahren hergestellten Stent ist zwischen der Oberfläche des metallischen oder keramischen Trägers und der Halbleiterverbindungsschicht eine Grenzschicht ausgebildet, die eine durch wechselseitige Erhebungen und Vertiefungen charakterisierte Mikrostruktur und eine legierungsartige chemische Struktur aufweist.

Die oberflächennnahe Schicht des vorgefertigten Stents bzw. des Trägermaterials und die Grenzschicht sind im wesentlichen frei von Sauerstoff und/oder Kohlenwasserstoffen, worauf die gute Haftung der Beschichtung zum Teil zurückzuführen ist.

Bei einer bevorzugten Ausführung ist die Grenzschicht zwischen der (metallischen oder keramischen) Oberfläche des vorgefertigten Stents und der Haftvermittlerschicht aus. dem Halbleitermaterial gebildet.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
Figur 1 eine schematische Querschnittsdarstellung des Schichtaufbaus bei einem gemäß einer Ausführungsform der Erfindung hergestellten Stent,
Figur 1a eine vergrößerte Ausschnittsdarstellung von Fig. 1,
Figur 2a bis 2c schematische Darstellungen zur Verdeutlichung des Verfahrenablaufes sowie einer Vorrichtung zur Herstellung eines Stents gemäß einer Ausführungsform der Erfindung und
Figur 3a und 3b eine schematische Darstellung des Potentialverlaufes zwischen den Elektroden der Vorrichtung in den Schritten (b) nach Fig. 2a und (d) nach Fig. 2d.

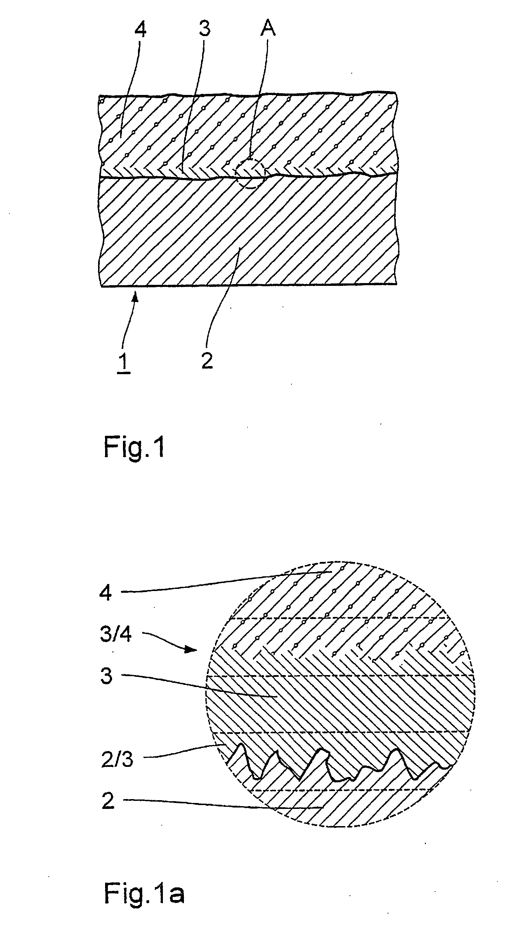

Figur 1 ist eine schematische (hinsichtlich der Schichtdickenverhältnisse nicht maßstäbliche) Querschnittsdarstellung des Schichtaufbaus bei einem antithrombogenen Stent 1 zur Anwendung in der Therapie koronarer Gefäßstenosen.

Auf einem Träger 2 aus 316L-Edelstahl ist eine Haftvermittlerschicht 3 aus amorphem Silizium a-Si mit einer Dicke von 3-5 nm und auf dieser eine Deckschicht 4 aus amorphem, n-dotiertem Siliziumkarbid a-SiC:H mit einer Dicke von 400 nm gebildet. Die Haftvermittlerschicht 3 und die Deckschicht 4 sind nicht durch eine definierte Grenzfläche voneinander getrennt, sondern gehen ineinander über.

Figur 1a zeigt in einer vergrößerten, aber ebenfalls schematischen Ausschnittsdarstellung von Fig. 1 den dort mit dem gestrichelten Kreis A umrandeten Ausschnitt. Hier ist zu erkennen, daß die Grenzfläche zwischen dem Träger 2 und der Haftvermittlerschicht 3 nicht eben, sondern in der Mikrostruktur stark zerklüftet ist, d.h. daß es eine (in der Figur durch gestrichelte Linien bezeichnete) Grenzschicht 2/3 mit ineinander verzahnten Erhebungen und Vertiefungen der benachbarten Materialien gibt, in der diese in einer legierungsähnlichen Struktur miteinander verschmolzen sind. Die Grenzfläche 3/4 der Haftvermittlerschicht 3 zur Deckschicht 4 weist eine ähnliche Mikrostruktur auf, wobei hier der Kohlenstoffanteil von unten nach oben ansteigt.

Von Bedeutung für ihre Funktion als Haftvermittlerschicht ist, daß es im Bereich der Haftvermittlerschicht einschließlich ihrer Grenzflächen praktisch keine Fremdatome, insbesondere keine oxidischen oder Kohlenwasserstoff-Anlagerungen an das Trägermaterial, gibt.

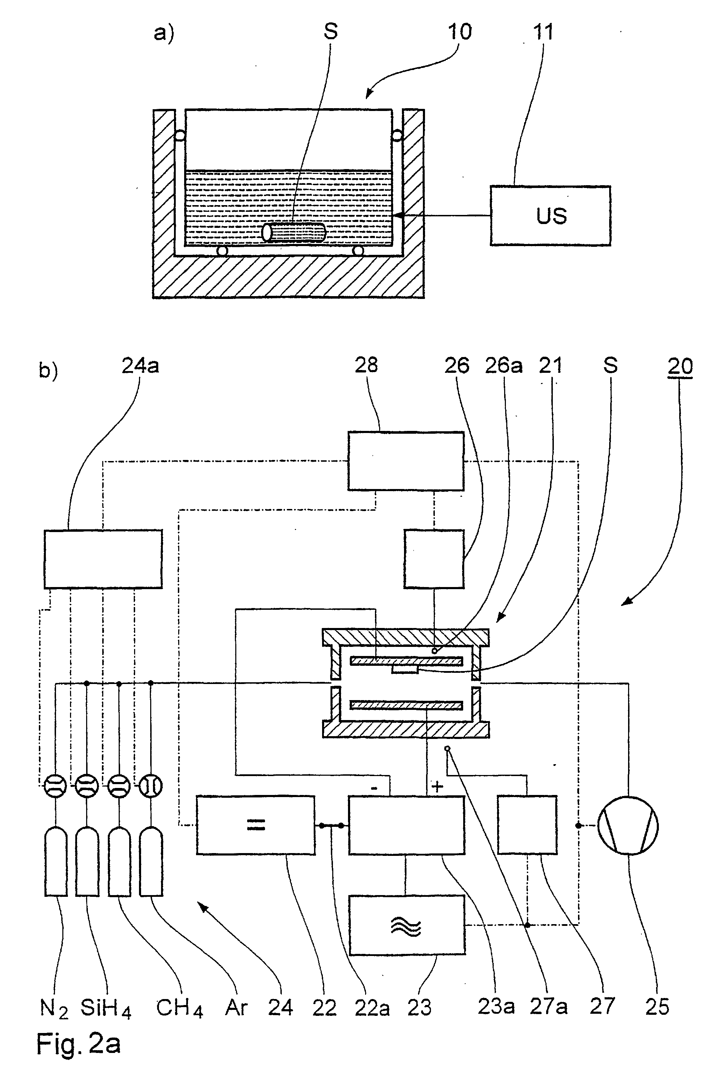

Die Figuren 2a bis 2c sind schematische Darstellungen zur Verdeutlichung der wesentlichen Schritte des Verfahrenablaufes bei der biokompatiblen, haftfesten Beschichtung eines Stents gemäß einer Ausführungsform der Erfindung.

Ein aus Edelstahl 316L vorgefertigter Basis-Stent S wird in einer ersten Verfahrensstufe (a) zunächst in einem Isopropanol-Bad 10 mittels Ultraschall gereinigt, der von einem herkömmlichen Ultraschallsender 11 geliefert wird.

In einer zweiten Stufe (b) wird der Basis-Stent S in einer Parallelplatten-Reaktoranlage 20 einer plasmaphysikalischen Ätzbehandlung unterzogen. Die Reaktoranlage 20 umfaßt neben einem eigentlichen Reaktorgefäß (Rezipienten) 21 eine Gleichspannungsquelle 22 mit Ausschalter 22a, einen HF-Sender 23 mit Anpassungsnetzwerk 23a, eine Gasversorgung 24 mit Durchflußregel- und -meßeinheit 24a, ein Vakuumerzeugungssystem 25, eine Druckmeßeinheit 26 mit Absolutdrucksensor 26a, eine Temperaturmeß- und -regeleinheit 27 mit pyroelektrischem T-Detektor 27a und eine Prozeßsteuereinheit 28.

In der praktischen Ausführung der Anlage umfaßt die Temperaturmeß- und regeleinheit 27 eine (in den Figuren nicht gezeigte) Gruppe von Halogenlampen, die auf die Stents gerichtet sind und eine nahezu trägheitslose Zusatzheizung bewirken können.

In der Stufe (b) werden zunächst der Rezipient auf einen Druck von unter 10⁻⁸ Bar evakuiert und die Stents für etwa 10 Minuten auf 250 °C vorgeheizt.

Anschließend wird in der Gasversorgung 24 nur das Ventil des Ar(Argon)-Behälters geöffnet, während die Ventile der übrigen Gasbehälter geschlossen sind. Durch die Gasversorgung 24 wird Argon in den Rezipienten eingelassen, wobei bei einem Gasfluß von bis zu etwa 40 sccm ein Druck im Bereich von 2x10⁻⁶ bis 10⁻⁵ Bar eingestellt wird.

Der Schalter 22a der Gleichstromversorgung 22 des Reaktors ist geschlossen, womit in der schematischen Darstellung das Anliegen einer (im Bereich von etwa 500 bis etwa 1500 Volt, vorzugsweise bei 1000 Volt, liegenden) negativen Biasspannung am Basis-Stent symbolisiert wird. Dieser ist also in dieser Verfahrensstufe kathodisch gepolt, wobei die Biasspannung sich nach den Anlagen- und den gewünschten Endprodukt-Parametern richtet. Über den HF-Sender 23 wird für die Dauer von 10 bis 15 min eine HF-Einkopplung mit einer Leistungsdichte im Bereich von 0,16 W/cm² in den Reaktorraum bzw. Rezipienten vorgenommen. Die Substrattemperatur wird bei 250 °C konstant gehalten

In dieser Phase findet unter den oben angegebenen Bedingungen ein Ionenätzen der Stent-Oberfläche statt, bei dem Anlagerungen - insbesondere von Kohlenwasserstoffen - wirkungsvoll entfernt werden und eine Erhöhung der Defektdichte an der Oberfläche stattfindet. Dadurch werden vorteilhafte Voraussetzungen für die Ausbildung stabiler chemischer Bindungen zwischen dem Träger (Substrat) und einer unmittelbar nachfolgend aufgebrachten Schicht geschaffen.

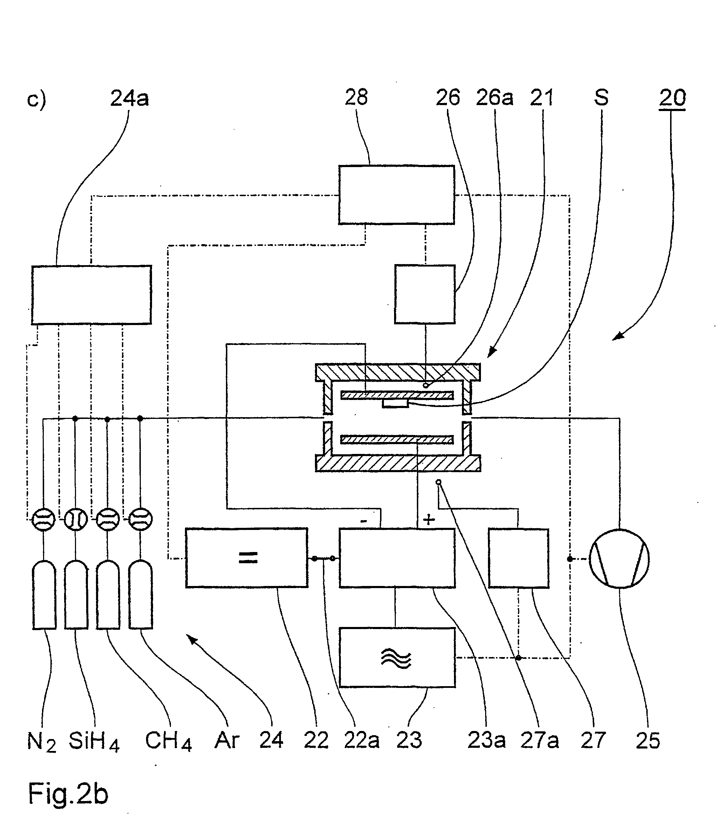

Die nächste Verfahrensstufe (c) wird - wie im oberen Teil von Fig. 2b schematisch gezeigt - ebenfalls in der Reaktoranlage 20 durchgeführt, wobei jetzt das Ventil des Ar-Behälters geschlossen und dasjenige des SiH₄-Behälters geöffnet ist. Der Gasfluß liegt bei 40 sccm und der Prozeßgasdruck bei 4x 10₋₅ Bar. Der Stent ist weiterhin mit einer negativen Vorspannung beaufschlagt, wobei die Biasspannung in dem in der Beschreibung zur Stufe (b) genannten Bereich oder vorzugsweise noch darüber (bei etwa 2000 V) liegt. Die Temperatur wird weiterhin bei 250 °C konstant gehalten.

In dieser Stufe findet ein hochenergetischer Beschuß der Stent-Oberfläche mit Silizium statt, das einerseits zu einer weiteren Strukturierung der Oberfläche und andererseits gleichzeitig zur Anlagerung von amorphem Silizium (a-Si) führt. Dies bildet nach einigen (etwa fünf) Minuten Prozeßdauer eine mit der Stent-Oberfläche eng verzahnte Schicht von 3-5 nm Dicke, in der Interdiffusionsprozesse entlang der Korngrenzen zur Bildung chemischer Verbindungen zwischen Bestandteilen des Substrats und dem Silizium führen.

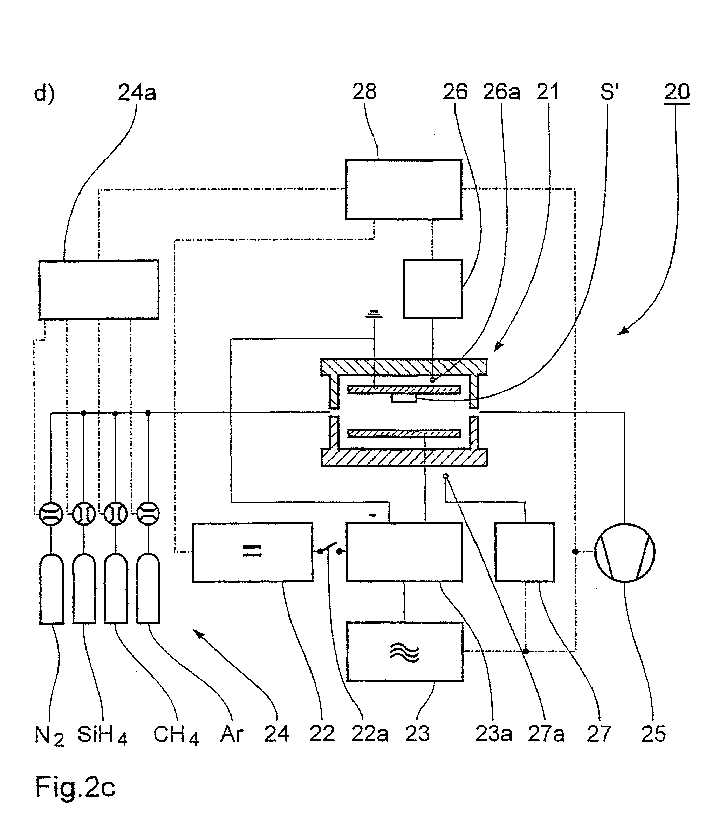

Die vierte wesentliche Verfahrensstufe (d) wird ebenfalls - wie in Fig. 2c unten zu erkennen - in der Reaktoranlage 20 durchgeführt, wobei jetzt in der Gasversorgung 24 die Ventile des SiH4- und des CH4-Behälters geöffnet sind. Die einzelnen Durchflußraten für Monosilan (SiH₄) und Methan (CH₄) - die in praxi in Mischung mit einer geringen Phosphin-Beigabe zugeführt werden - werden entsprechend den gewünschten Elementanteilen an Si und C in der abzuscheidenden Schicht durch die Prozeßsteuereinheit 28 individuell gesteuert. Als eine vorteilhafte Einstellung der Gasflüsse haben sich 35,5 sccm für SiH₄, 3,53 sccm für CH₄ und 37,2 sccm für PH₃ ergeben. Der Prozeßgasdruck beträgt 8x10⁻⁵ Bar und die Substrattemperatur wiederum 250 °C. Sie kann in dieser Verfahrensstufe nur durch die Halogen-Zusatzheizung erreicht werden, da keine Erwärmung durch einen Ionenbeschuß mehr erfolgt.

Die die Stents (Substrate) aufnehmende Elektrode ist jetzt geerdet, und es liegt keine Gleichspannung mehr an den Elektroden an (was in der Figur durch Öffnung des Schalters 22a symbolisiert ist), d.h. dieser Schritt (d) wird bei nicht vorgespanntem Substrat mit kapazitiv eingekoppelter, von der HF-Stufe 23 gelieferter HF von 13,56 MHz mit einer Leistungsdichte von 0,16 W/cm² durchgeführt.

Im Zuge des - als solches bekannten - PECVD-Verfahrens wird in einer SiH₄-/CH₄-Atmosphäre mit über die Ventile der Gasversorgung 24 vorbestimmter Gaszusammensetzung eine einige hundert - vorzugsweise etwa 400 - nm dicke Schicht aus amorphem Siliziumkarbid (a-SiC) auf dem Edelstahl-Stent S (genauer gesagt: auf der diesen bedeckenden dünnen a-Si-Schicht) abgeschieden. Da die Zuführung der Monosilans bei Öffnung des entsprechenden Ventils der Gasversorgung 24 nicht schlagartig erfolgt, gehen die Schicht aus reinem Si und die SiC-Schicht unter gradueller Zunahme des C-Anteils ineinander über. Damit ist ein biokompatibler Stent S' fertiggestellt.

Die biokompatible SiC-Schicht hat eine ausgezeichnete Haftfestigkeit, die sich in einer wesentlich erhöhten kritischen Kraft beim üblichen Scratch-Test äußert.

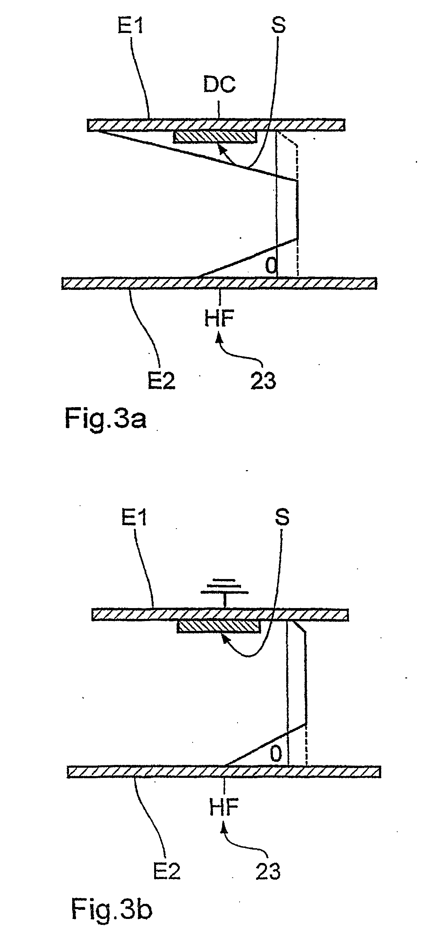

Die Figuren 3a und 3b sind schematische Darstellungen des Potentialverlaufes zwischen den Elektroden der Vorrichtung in den Schritten (b) nach Fig. 2a und (d) nach Fig. 2c. Die obere Elektrode E1 trägt das Substrat, an die untere Elektrode E2 wird kapazitiv die HF von der HF-Stufe 23 eingekoppelt. In den Stufen (b) und (c) (Fig. 3a) liegt an der oberen Elektrode E1 eine hohe negative Vorspannung an, in Stufe (d) (Fig. 3b) ist diese Elektrode geerdet.

Durch den in Fig. 3a dargestellten Potentialverlauf werden die Prozeßgasteilchen sehr effektiv ionisiert und hohe Ionenströme auf die Oberfläche des Basis-Stents beschleunigt, die zu einem wirksamen Abtrag von Verunreinigungen führen. Beim Übergang zur Beschichtung ist gemäß Fig. 3b lediglich die Gleichspannung auszuschalten, wobei das Plasma erhalten bleibt und Unterbrechungen des Prozeßablaufes, die in nachteiliger Weise zur Anlagerung von Restgasen an die Oberfläche und somit zu einer Verschlechterung der Schichthaftung führen würden, nicht auftreten.

Die Realisierung der gezeigten Potentialverläufe erfordert den Einsatz von Filtern, die einerseits eine Einkopplung der HF in die Gleichspannungsquelle und andererseits eine gegenseitige Beeinflussung zwischen Gleichspannungsquelle 22 und HF-Generator 23 verhindern. Dies sind im Anpassungsnetzwerk 23a enthalten.

Die gezeigte Gestalt und Lage der Komponenten der Anlage 20 sowie auch der Lage des Stents S in dieser ist nur im Sinne einer rein schematischen Darstellung zu verstehen. Die Anlage kann vielfältig modifiziert sein, wobei bei anderem Aufbau ggfs. auch wesentlich veränderte Leistungsdichten und Gasflüsse zur Bildung einer optimalen Schicht eingestellt werden müssen.

Das oben beschriebene Verfahren ist mit entsprechend variierten Materialien und Verfahrensparametern auch für andere Implantatwerkstoffe und zu deren Beschichtung mit anderen biokompatiblen Schichten nutzbar. Insbesondere ist in ähnlicher Weise eine Beschichtung von Titanlegierungen (etwa TiAl5Fe2,5), Tantal, Platin/Iridium, pyrolytischem Kohlenstoff oder Oxidkeramik (etwa ZrO₂) mit a-SiC möglich. Grundsätzlich sind auch andere halbleitende Beschichtungsmaterialien mit geeigneter Bandlücke einsetzbar, wobei sich die Wahl der Prozeßgase dann natürlich nach der chemischen Zusammensetzung der zu erzeugenden Schicht richtet, für eine bestimmte Schicht-Zusammensetzung aber als grundsätzlich bekannt anzusehen ist. SiC ist jedoch nach den Untersuchungen der Erfinder unter mehreren Aspekten als vorteilhaftes Material anzusehen.

Der Schritt des Aufbringens der Zwischenschicht kann auch weggelassen werden; als erfindungswesentlich ist die Ausführung der Verfahrensstufen (b) und (d) gemäß Fig. 2a und 2c anzusehen.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch macht.

## Patentansprüche

1. Verfahren zur Herstellung eines nichtkollabierenden intravasalen Stents (S) im wesentlichen aus einem metallischen oder keramischen Material (2); **gekennzeichnet durch** die Schritte:
a) Einbringen des vorgefertigten Stents (S) in den Rezipienten (21) einer CVD-Anlage (20) und Evakuierung desselben auf einen vorbestimmten Druck,
b) Anlegen einer vorbestimmten negativen Biasspannung an den vorgefertigten Stent, Zuführen eines Ätzgases mit einer vorbestimmten ersten Flussrate und Einkoppeln von HF-Leistung mit einer ersten vorbestimmten Leistungsdichte in den Rezipienten (21) zur Ausführung eines plasmagestützten lonenätzens oder reaktiven lonenätzens für eine erste vorbestimmte Zeitdauer zur Oberflächenbehandlung des Stents,
c) Unterbrechung der Ätzgaszuführung, Aufrechterhalten der negativen Biasspannung am Stent und hochenergetischer Beschuss der Stent-Oberfläche mit einem ein Halbleiter-Element in gebundener Form enthaltenden Prozessgas, wobei eine Haftvermittlerschicht (3) mit einer Grenzschicht (2/3) mit einer **durch** wechselseitige Erhebungen und Vertiefungen charakterisierten Mikrostruktur abgeschieden wird und
d) Trennung des oberflächenbehandelten Stents von der negativen Biasspannung, Zuführen eines ein Halbleiter-Element in gebundener Form enthaltenden, mehrkomponentigen Gemisches aus Prozessgasen mit vorbestimmten zweiten Flussraten und Einkoppeln von HF-Leistung mit einer vorbestimmten zweiten Leistungsdichte in den Rezipienten für eine zweite vorbestimmte Zeitdauer zur Ausführung einer plasmaunterstützten chemischen Gasphasenabscheidung (PECVD) einer biokompatiblen Halbleiterverbindungsschicht (4).

2. Verfahren nach Anspruch 1, **dadurch gekennzeichnet, dass** nach dem Schritt der Oberflächenbehandlung des vorgefertigten Stents ein Schritt
- Anlegen einer vorbestimmten zweiten negativen Biasspannung an den oberflächenbehandelten Stent, Zuführen eines das Halbleitermaterial in gebundener Form enthaltenden Prozessgases mit einer vorbestimmten dritten Flussrate und Einkoppeln von HF-Leistung mit einer dritten vorbestimmten Leistungsdichte in den Rezipienten (21) für eine vorbestimmte dritte Zeitdauer zur Ausführung einer plasmagestützten Abscheidung einer dünnen, das Halbleiterelement aufweisenden Haftvermittlerschicht (3) auf den Stent ausgeführt wird.

3. Verfahren nach Anspruch 1 oder 2, **dadurch gekennzeichnet, dass** das Halbleiter-Element Silizium ist und die Halbleiterverbindungsschicht (4) Siliziumkarbid ausweist.

4. Verfahren nach einem der vorangehenden Ansprüche, **dadurch gekennzeichnet dass** die Bildung der Halbleiterverbindungsschicht (4) als amorphe Schicht erfolgt.

5. Verfahren nach einem der vorangehenden Ansprüche, **dadurch gekennzeichnet, dass** das Ätzgas im Schritt der Oberflächenbehandlung ein Edelgas, Insbesondere Argon oder Tetrachlorkohlenstoff ist.

6. Verfahren nach einem der vorangegangenen Ansprüche **dadurch gekennzeichnet, dass** das Gemisch aus Prozessgasen im Schritt der Gasphasenabscheidung Monosilan sowie Methan aufweist.

7. Verfahren nach einem der Ansprüche 1 bis 6, **dadurch gekennzeichnet, dass** die Haftvermittlerschicht im wesentlichen aus Silizium besteht.

8. Verfahren nach Anspruch 7, **dadurch gekennzeichnet, dass** das Prozessgas im Schritt der Bildung der Haftvermittlerschicht Monosilan in Wasserstoff aufweist.

9. Verfahren nach einem der vorangehenden Ansprüche, **dadurch gekennzeichnet, dass** ein vorgefertigter Stent aus Edelstahl oder einer Titanlegierung verwender wird.

10. Verfahren nach einem der vorangehenden Ansprüche, **dadurch gekennzeichnet, dass** die Temperatur des vorgefertigten Stents während des gesamten Prozessablaufes konstant, vorzugsweise auf im wesentlichen 250 °C, gehalten wird.

11. Verfahren nach Anspruch 10, **dadurch gekennzeichnet, dass** beim Übergang zum Schritt der Gasphasenabscheidung und bei Ausführung dieses Schritts der Stent über eine im wesentlichen trägheitslose Substratheizung beheizt wird.

12. Verfahren nach einem der vorangehenden Ansprüche, **dadurch gekennzeichnet, dass** die Trennung des Stents von der negativen Biasspannung ohne Unterbrechung der Einkopplung der HF-Leistung und somit unter Aufrechterhaltung des Plasmas Im Rezipienten (21) erfolgt.

13. Verfahren nach einen der vorangehenden Ansprüche, **dadurch gekennzeichnet, dass** die Hatbleiterverbindungsschicht (4) mit einer Dicke von 400 nm aufgebracht wird.

14. Verfahren nach einem der Ansprüche 2 bis 13, **dadurch gekennzeichnet, dass** die Haftvermittlerschicht (3) mit einer Dicke von 3 bis 5 nm gebildet wird.

15. Verfahren nach einem der Ansprüche 2 bis 14, **dadurch gekennzeichnet, dass** die Haftvermittlerschicht (3) eine kontinuierliche Veränderung der Prozessgaszusammensetzung über einen vorbestimmten Zeitraum ohne ausgeprägte Grenzfläche unter Abnahme des Anteils des Halblelterelementes mit zunehmendem Abstand von der Oberfläche des vorgefertigten Stents ineinander übergehend gebildet werden.

16. Verfahren nach einem der vorangehenden Ansprüche, **dadurch gekennzeichnet, dass** das Ätzgas ein einkomponentiges Ätzgas ist.

17. Verfahren nach einem der Ansprüche 2 bis 16, **dadurch gekennzeichnet, dass** das nach dem Anlegen einer zweiten negativen Biasspannung zugeführte Prozessgas ein einkomponentiges Prozessgas ist.

18. Nichtkollabierender intravasaler Stent mit einem metallischen oder keramischen Grundkörper (2), dessen Oberfläche mit einer biokompatiblen Halbleiterverbindungsschicht (4) bedeckt ist, **gekennzeichnet durch** eine aus einem Halbleitermaterial gebildeten Haftvermittterschicht (3) mit einer Grenzschicht (2/3) zum Grundkörper (2) hin, wobei die Grenzschicht (2/3) **durch** wechselseitige Erhebungen und Vertiefungen charakterisierte Mikrostruktur aufweist und darauf die biokompatible Halbleiterverbindungsschicht (4) abgeschieden ist.

19. Stent nach Anspruch 18, **dadurch gekennzeichnet, dass** die Grenzschicht zwischen der Oberfläche des vorgefertigten Stents (S; 2) und einer auf diese aufgebrachten Haftvermittlerschicht (3) aus dem Halbleitermaterial gebildet ist.

20. Stent nach Anspruch 18 oder 19, **dadurch gekennzeichnet, dass** die Grenzschicht (2/3) eine legierungsartige chemische Struktur aufweist.

21. Stent nach einem der Ansprüche 18 bis 20, **dadurch gekennzeichnet, dass** die Oberfläche des vorgefertigten Stents (S; 2) und die Grenzschicht (2/3) im wesentlichen frei von Sauerstoff und/oder Kohlenwasserstoff sind.

## Claims

1. A process for producing a non-collapsible intravascular stent (S) substantially comprising a metal or ceramic material (2); **characterised by** the following steps:
a) introducing the prefabricated stent (S) into the container (21) of a CVD-installation (20) and evacuating same to a predetermined pressure,
b) applying a predetermined negative bias voltage to the prefabricated stent, supplying an etching gas at a predetermined first flow rate and coupling RF-power at a first predetermined power density into the container (21) for effecting a plasma-supported ion etching operation or reactive ion etching operation for a first predetermined period of time for the surface treatment of the stent,
c) interrupting the supply of etching gas, maintaining the negative bias voltage at the stent and high-energy bombardment of the stent surface with a process gas containing a semiconductor element in bound form, wherein a bonding layer (3) with an interface (2/3) with a microstructure **characterised by** alternate raised portions and recesses is deposited, and
d) separating the surface-treated stent from the negative bias voltage, supplying a multi-component mixture of process gases, which contains a semiconductor element in bound form, at a predetermined second flow rate and coupling RF-power at a predetermined second power density into the container for a second predetermined period of time for effecting plasma-supported chemical gaseous phase deposition (PECVD) of a biocompatible semiconductor connecting layer (4).

2. A process according to claim 1 **characterised in that** after the step of surface treatment of the prefabricated stent, a step is effected comprising:
- applying a predetermined second negative bias voltage to the surface-treated stent, supplying a process gas containing the semiconductor material in bound form, at a predetermined third flow rate and coupling RF-power at a third predetermined power density into the container (21) for a predetermined third period of time for effecting plasma-supported deposition of a thin bonding layer (3) having the semiconductor element on the stent.

3. A process according to claim 1 or claim 2 **characterised in that** the semiconductor element is silicon and the semiconductor connecting layer (4) has silicon carbide.

4. A process according to one of the preceding claims **characterised in that** formation of the semiconductor connecting layer (4) is effected in the form of an amorphous layer.

5. A process according to one of the preceding claims **characterised in that** the etching gas in the surface treatment step is a noble gas, in particular argon or carbon tetrachloride.

6. A process according to one of the preceding claims **characterised in that** the mixture of process gases in the gaseous phase deposition step has monosilane and methane.

7. A process according to one of claims 1 to 6 **characterised in that** the bonding layer substantially comprises silicon.

8. A process according to claim 7 **characterised in that** the process gas in the step of forming the bonding layer has monosilane in hydrogen.

9. A process according to one of the preceding claims **characterised in that** a prefabricated stent of high-quality steel or a titanium alloy is used.

10. A process according to one of the preceding claims **characterised in that** the temperature of the prefabricated stent is kept constant, preferably at substantially 250°C, throughout the entire operating procedure.

11. A process according to claim 10 **characterised in that**, in the transition to the step of gaseous phase deposition and when effecting that step the stent is heated by way of a substantially inertia-less substrate heating means.

12. A process according to one of the preceding claims **characterised in that** separation of the stent from the negative bias voltage is effected without interrupting the coupling-in of the RF-power and thus while maintaining the plasma in the container (21).

13. A process according to one of the preceding claims **characterised in that** the semiconductor connecting layer (4) is applied in a thickness of 400 nm.

14. A process according to one of claims 2 to 13 **characterised in that** the bonding layer (3) is formed in a thickness of 3 to 5 nm.

15. A process according to one of claims 2 to 14 **characterised in that** the bonding layer (3) is formed by a continuous variation in the process gas composition over a predetermined period of time without a pronounced interface with a reduction in the proportion of the semiconductor element with increasing spacing from the surface of the prefabricated stent in a mutually merging condition.

16. A process according to one of the preceding claims **characterised in that** the etching gas is a single-component etching gas.

17. A process according to one of claims 2 to 16 **characterised in that** the process gas supplied after the application of a second negative bias voltage is a single-component process gas.

18. A non-collapsible intravascular stent with a metallic or ceramic main body (2) whose surface is covered with a biocompatible semiconductor connecting layer (4), **characterised by** a bonding agent (3) formed from a semiconductor material and having an interface (2/3) towards the main body (2), wherein the interface (2/3) has a microstructure **characterised by** alternate raised portions and recesses and the biocompatible semiconductor connecting layer (4) is deposited thereon.

19. A stent according to claim 18 **characterised in that** the interface between the surface of the prefabricated stent (S; 2) and a bonding layer (3) applied to said surface is formed from the semiconductor material.

20. A stent according to claim 18 or claim 19 **characterised in that** the interface (2/3) is of an alloy-like chemical structure.

21. A stent according to one of claims 18 to 20 **characterised in that** the surface of the prefabricated stent (S; 2) and the interface (2/3) are substantially free of oxygen and/or hydrocarbon.

## Revendications

1. Procédé de fabrication d'un extenseur intravasculaire qui ne s'affaisse pas (S) essentiellement en un matériau métallique ou céramique (2), **caractérisé par** les étapes suivantes :
a) introduction de l'extenseur (S) préfabriqué dans le récipient (21) d'une installation de CVD (20) et mise sous vide de celui-ci à une pression prédéterminée ;
b) application d'une tension de polarisation négative prédéterminée à l'extenseur préfabriqué, introduction d'un gaz d'attaque à un premier débit prédéterminé et couplage de puissance HF ayant une première puissance volumique prédéterminée dans le récipient (21) pour la mise en oeuvre d'une attaque ionique assistée par plasma ou d'une attaque ionique réactive pendant une première durée prédéterminée pour le traitement de surface de l'extenseur ;
c) interruption de l'introduction du gaz d'attaque, maintien de la tension de polarisation négative sur l'extenseur et bombardement à haute énergie de la surface de l'extenseur avec un gaz de traitement contenant un élément semiconducteur sous forme liée, de sorte qu'une couche de promoteur d'adhésion (3), ayant une couche limite (2/3) comportant une microstructure, **caractérisée par** des protubérances et des évidements réciproques est déposée et
d) séparation de l'extenseur traité en surface d'avec la tension de polarisation négative, introduction d'un mélange de gaz de traitement à plusieurs composants contenant un élément semiconducteur sous forme liée à des seconds débits prédéterminés et couplage de puissance HF ayant une seconde puissance volumique prédéterminée dans le récipient pendant une seconde durée prédéterminée pour la mise en oeuvre d'un dépôt chimique en phase vapeur assisté par plasma (PECVD) d'une couche de composé semiconducteur biocompatible (4).

2. Procédé selon la revendication 1, **caractérisé en ce que**, après l'étape de traitement en surface de l'extenseur préfabriqué, une étape
- d'application d'une seconde tension de polarisation négative prédéterminée à l'extenseur traité en surface, d'introduction d'un gaz de traitement contenant le matériau semiconducteur sous forme liée à un troisième débit prédéterminé et de couplage de puissance HF ayant une troisième puissance volumique prédéterminée dans le récipient (21) pendant une troisième durée prédéterminée pour la mise en oeuvre d'un dépôt assisté par plasma sur l'extenseur d'une mince couche de promoteur d'adhésion (3) comportant l'élément semiconducteur
est mise en oeuvre.

3. Procédé selon la revendication 1 ou 2, **caractérisé en ce que** l'élément semiconducteur est le silicium et la couche de composé semiconducteur (4) comporte du carbure de silicium.

4. Procédé selon l'une des revendications précédentes, **caractérisé en ce que** la formation de la couche de composé semiconducteur (4) se déroule sous forme de couche amorphe.

5. Procédé selon l'une des revendications précédentes, **caractérisé en ce que** le gaz d'attaque dans l'étape de traitement de la surface est un gaz noble, en particulier l'argon, ou le tétrachlorure de carbone.

6. Procédé selon l'une des revendications précédentes, **caractérisé en ce que** le mélange de gaz de traitement dans l'étape de dépôt en phase vapeur comporte du monosilane ainsi que du méthane.

7. Procédé selon l'une des revendications 1 à 6, **caractérisé en ce que** la couche de promoteur d'adhésion consiste essentiellement en silicium.

8. Procédé selon la revendication 7, **caractérisé en ce que** le gaz de traitement dans l'étape de formation de la couche de promoteur d'adhésion comporte du monosilane dans l'hydrogène.

9. Procédé selon l'une des revendications précédentes, **caractérisé en ce qu'**un extenseur préfabriqué en acier spécial ou en un alliage du titane est utilisé.

10. Procédé selon l'une des revendications précédentes, **caractérisé en ce que** la température de l'extenseur préfabriqué est maintenue constante pendant tout le déroulement du procédé, de préférence sensiblement à 250°C.

11. Procédé selon la revendication 10, **caractérisé en ce que** l'extenseur est chauffé par le biais d'un dispositif de chauffage de substrat sensiblement sans inertie lors du passage à l'étape de dépôt en phase vapeur et lors de la mise en oeuvre de cette étape.

12. Procédé selon l'une des revendications précédentes, **caractérisé en ce que** la séparation de l'extenseur d'avec la tension de polarisation négative a lieu sans interruption du couplage de la puissance HF et donc avec maintien du plasma dans le récipient (21).

13. Procédé selon l'une des revendications précédentes, **caractérisé en ce que** la couche de composé semiconducteur (4) est appliquée en une épaisseur de 400 nm.

14. Procédé selon l'une des revendications 2 à 13, **caractérisé en ce que** la couche de promoteur d'adhésion (3) est formée en une épaisseur de 3 à 5 nm.

15. Procédé selon l'une des revendications 2 à 14, **caractérisé en ce que** la couche de promoteur d'adhésion (3) et la couche de composé semiconducteur sont formées de manière mutuellement pénétrante par une modification continue de la composition du gaz de traitement pendant une durée prédéterminée sans surface limite marquée avec diminution de la proportion de l'élément semiconducteur quand la distance avec la surface de l'extenseur préfabriqué augmente.

16. Procédé selon l'une des revendications précédentes, **caractérisé en ce que** le gaz d'attaque est un gaz d'attaque à un composant.

17. Procédé selon l'une des revendications 2 à 16, **caractérisé en ce que** le gaz de traitement introduit après l'application d'une seconde tension de polarisation négative est un gaz de traitement à un composant.

18. Extenseur intravasculaire qui ne s'affaisse pas comportant un corps de base (2) métallique ou céramique dont la surface est recouverte d'une couche de composé semiconducteur (4) biocompatible, **caractérisé par** une couche de promoteur d'adhésion (3) formée à partir d'un matériau semiconducteur ayant une couche limite (2/3) avec le corps de base (2), la couche limite (2/3) comportant une microstructure **caractérisée par** des protubérances et des évidements réciproques et la couche de composé semiconducteur (4) biocompatible étant déposée sur celle-ci.

19. Extenseur selon la revendication 18, **caractérisé en ce que** la couche limite entre la surface de l'extenseur préfabriqué (S ; 2) et une couche de promoteur d'adhésion (3) appliquée sur celle-ci est formée par le matériau semiconducteur.

20. Extenseur selon la revendication 18 ou 19, **caractérisé en ce que** la couche limite (2/3) présente une structure chimique analogue à un alliage.

21. Extenseur selon l'une des revendications 18 à 20, **caractérisé en ce que** la surface de l'extenseur préfabriqué (S ; 2) et la couche limite (2/3) sont sensiblement dépourvues d'oxygène et/ou d'hydrocarbure.
